# EUROPEAN PATENT SPECIFICATION

(11) **EP 1 353 687 B1**
(45) Date of publication and mention of the grant of the patent: **26.07.2006**
(21) Application number: 01992581.7
(22) Date of filing: 29.10.2001
(51) Int. Cl.: A61K 38/17, A61K 39/395, A61P 37/00, A61P 31/00, A61P 35/00

(54) **METHODS FOR OBTAINING INHIBITORS OF TIRC7 LIGAND BINDING AND USES THEREOF**
VERFAHREN ZUR GEWINNUNG VON INHIBITOREN DER TIRC7-LIGANDEN-BINDUNG UND VERWENDUNGSZWECKE DAFÜR
METHODES POUR OBTENIR DES AGENTS INHIBITEURS DE LA LIAISON DE LA TIRC7 A SON LIGAND, ET UTILISATIONS CORRESPONDANTES

(30) Priority: 30.10.2000 EP 00123666
(43) Date of publication of application: 22.10.2003
(73) Proprietor: GenPat77 Pharmacogenetics AG, 10115 Berlin (DE)
(72) Inventor: Utku, Nalan, 10719 Berlin (DE)
(74) Representative: Müller Fottner Steinecke
(86) International application number: PCT/EP2001/012485
(87) International publication number: WO 2002/036149

(56) References cited:
- WO-A-99/11782
- UTKU N ET AL: "PREVENTION OF ACUTE ALLOGRAFT REJECTION BY ANTIBODY TARGETING OF TIRC7, A NOVEL T CELL MEMBRANE PROTEIN" IMMUNITY, CELL PRESS, US, vol. 9, October 1998 (1998-10), pages 509-518, XP002188110 ISSN: 1074-7613 cited in the application
- SAUBERMANN L J ET AL: "TIRC7 SPECIFIC AB, A NOVEL INHIBITOR OF ACTIVATED INTESTINAL T CELLS IN VITRO" GASTROENTEROLOGY, SAUNDERS, PHILADELPHIA, PA,, US, vol. 4, PART 2, no. 116, April 1999 (1999-04), page A812 XP001073583 ISSN: 0016-5085

### SEQUENCE LISTING

<110> GenPat 77 Pharmacogenetics AG
<120> Use of inhibitors of TIRC7 ligand binding
<130> E 2385 PCT
<140>
   <141>
<150> EP 0012 3666.0
   <151> 2001-10-30
<160> 2
<170> PatentIn Ver. 2.1
<210> 1
   <211> 819
   <212> DNA
   <213> Homo sapiens
<400> 1
   actcccaaaa gagcgcccaa gaagaaaatg gccataagtg gagtccctgt gctaggattt 60
   ttcatcatag ctgtgctgat gagcgctcag gaatcatggg ctatcaaaga agaacatgtg 120
   atcatccagg ccgagttcta tctgaatcct gaccaatcag gcgagtttat gtttgacttt 180
   gatggtgatg agattttcca tgtggatatg gcaaagaagg agacggtctg gcggcttgaa 240
   gaatttggac gatttgccag ctttgaggct caaggtgcat tggccaacat agctgtggac 300
   aaagccaact tggaaatcat gacaaagcgc tccaactata ctccgatcac caatgtacct 360
   ccagaggtaa ctgtgctcac gaacagccct gtggaactga gagagcccaa cgtcctcatc 420
   tgtttcatag acaagttcac cccaccagtg gtcaatgtca cgtggcttcg aaatggaaaa 480
   cctgtcacca caggagtgtc agagacagtc ttcctgccca gggaagacca ccttttccgc 540
   aagttccact atctcccctt cctgccctca actgaggacg tttacgactg cagggtggag 600
   cactggggct tggatgagcc tcttctcaag cactgggagt ttgatgctcc aagccctctc 660
   ccagagacta cagagaacgt ggtgtgtgcc ctgggcctga ctgtgggtct ggtgggcatc 720
   attattggga ccatcttcat catcaaggga ttgcgcaaaa gcaatgcagc agaacgcagg 780
   gggcctctgt aaggcacatg gaggtgatgg tgtttctta                       819
<210> 2
   <211> 254
   <212> PRT
   <213> Homo sapiens
<400> 2
   Met Ala Ile Ser Gly Val Pro Val Leu Gly Phe Phe Ile Ile Ala Val
   1               5                   10                  15
   Leu Met Ser Ala Gln Glu Ser Trp Ala Ile Lys Glu Glu His Val Ile
               20                  25                  30
   Ile Gln Ala Glu Phe Tyr Leu Asn Pro Asp Gln Ser Gly Glu Phe Met
           35                  40                  45
   Phe Asp Phe Asp Gly Asp Glu Ile Phe His Val Asp Met Ala Lys Lys
           50                  55                  60
   Glu Thr Val Trp Arg Leu Glu Glu Phe Gly Arg Phe Ala Ser Phe Glu
    65                      70                  75                  80
   Ala Gln Gly Ala Leu Ala Asn Ile Ala Val Asp Lys Ala Asn Leu Glu
                        85                  90                  95
   Ile Met Thr Lys Arg Ser Asn Tyr Thr Pro Ile Thr Asn Val Pro Pro
               100                 105                 110
   Glu Val Thr Val Leu Thr Asn Ser Pro Val Glu Leu Arg Glu Pro Asn
           115                 120                 125
   Val Leu Ile Cys Phe Ile Asp Lys Phe Thr Pro Pro Val Val Asn Val
       130                 135                 140
   Thr Trp Leu Arg Asn Gly Lys Pro Val Thr Thr Gly Val Ser Glu Thr
   145                 150                 155                 160
   Val Phe Leu Pro Arg Glu Asp His Leu Phe Arg Lys Phe His Tyr Leu
                   165                 170                 175
   Pro Phe Leu Pro Ser Thr Glu Asp Val Tyr Asp Cys Arg Val Glu His
               180                 185                 190
   Trp Gly Leu Asp Glu Pro Leu Leu Lys His Trp Glu Phe Asp Ala Pro
           195                 200                 205
   Ser Pro Leu Pro Glu Thr Thr Glu Asn Val Val Cys Ala Leu Gly Leu
       210                 215                 220
   Thr Val Gly Leu Val Gly Ile Ile Ile Gly Thr Ile Phe Ile Ile Lys
   225                 230                 235                 240
   Gly Leu Arg Lys Ser Asn Ala Ala Glu Arg Arg Gly Pro Leu
                   245                 250

## Description

The present invention relates to the use of an inhibitor interfering with the interaction of TIRC7 with its ligand(s) for the preparation of a pharmaceutical composition for the treatment of graft versus host disease, autoimmune diseases, allergic diseases, infectious diseases, sepsis, for the treatment of tumors, for the improvement of wound healing or for inducing or maintaining immune unresponsiveness in a subject. In addition, the present invention relates to the use of a nucleic acid molecule encoding said inhibitor and of a vector comprising said nucleic acid molecule for the preparation of a pharmaceutical composition for amelioration, treatment and prevention of immunological diseases. Additionally, the present invention provides methods of identifying an agent capable of interfering with the interaction between TIRC7 and its ligand and/or of identifying a (poly)peptide involved in the regulation of the immune response in a subject. In a further embodiment, the present invention relates to a method of refining an inhibitor, an agent or a (poly)peptide as defined herein or identified by the method(s) of the invention. Furthermore, the invention relates to the preparation of pharmaceutical compositions comprising the agents, (poly)peptides identified and/or refined by the methods as disclosed herein. In a further embodiment, the present invention relates to the use of an inhibitor, an agent or a (poly)peptide as identified and/or refined by the methods as disclosed herein for the preparation of a pharmaceutical composition.

Several documents are cited throughout the text of this specification.

There is no admission that any document cited is indeed prior art as to the present invention. T-cell activation is a serial process involving multiple signaling pathways and sequential changes in gene expression resulting in differentiation of T-cells into distinct subpopulations, i.e. Th1 and Th2, which are distinguishable by their pattern of cytokine production and characterize the mode of cellular immune response. The T-cell response is initiated by the interaction of the antigen-specific T-cell receptor (TCR) with peptide presented by major histocompatibility complex (MHC) molecules on the surface of antigen presenting cells (APCs). Additional signals are provided by a network of receptor-ligand interactions mediated by a number of membrane proteins such as CD28/CTLA4 and B7, CD40/CD40L, LFA-1 and ICAM-1 (Lenschow, Science 257 (1992), 789-792; Linsley, Annu. Rev. Immunol. 11 (1993), 191-212; Xu, Immunity 1 (1994), 423-431; Bachmann, Immunity 7 (1997), 549-557; Schwartz, Cell 71 (1992), 1065-1068) collectively called costimulatory signals (Perez, Immunity 6 (1997), 411). These membrane proteins can alter T-cell activation in distinct ways (Bachmann, Immunity 7 (1997), 549-557) and regulate the immune response by the integration of positive and negative signals provided by these molecules (Bluestone, Immunity 2 (1995), 555-559; Perez, Immunity 6 (1997), 411). Many of the agents which are effective in modulating the cellular immune response either interfere with the T-cell receptor (Cosimi, Transplantation 32 (1981), 535-539), block costimulatory signaling (Larsen, Nature 381 (1996), 434-438; Blazar J. Immune. 157 (1996), 3250-3259; Kirk, Proc. Natl. Acad. Sci. USA 94 (1997), 8789-8794; Linsley, Science 257 (1992), 792-95; Turka, Proc. Natl. Acad. Sci. USA 89 (1992), 11102-11105) or inhibit intracellular activation signals downstream from these primary cell membrane triggers (Schreiber and Crabtree, Immunology Today 13 (1992), 136-42). Therapeutic prevention of T-cell activation in organ transplantation and autoimmune diseases presently relies on panimmunosupressive drugs interfering with downstream intracellular events. Specific modulation of the immune response remains a longstanding goal in immunological research.

In view of the need of therapeutic means for the treatment of diseases related to immune responses of the human body, the technical problem of the present invention is to provide for means and methods for modulation of the immune response such as immune unresponsiveness in a subject. The solution to said technical problem is achieved by providing the embodiments characterized in the claims.

Accordingly, in one aspect the present Invention relates to the use of an inhibitor interfering with the interaction of TIRC7 with its ligand for the preparation of a pharmaceutical composition for the treatment of graft versus host disease, autoimmune diseases, allergic diseases, infectious diseases, sepsis, for the treatment of tumors, for the improvement of wound healing or for inducing or maintaining immune unresponsiveness in a subject.

The term "TIRC7" as used in accordance with the present invention, denotes a protein involved in the signal transduction of T-cell activation and/or proliferation and that, preferably in a soluble form is capable of inhibiting or suppressing T-cell proliferation in response to alloactivation in a mixed lymphocyte culture or in response to mitogens when exogeneously added to the culture. *In vitro* translated TIRC7 protein is able to efficiently suppress in a dose dependent manner the proliferation of T-celis in response to alloactivation in a mixed lymphocyte culture or in response to mitogens. TIRC7 is known to the person skilled in the art and described, inter alia, in WO99/117 and in Utku et al., Immunity 9 (1998), 509-518.

The term "inhibitor interfering with the interaction of TIRC7 with its ligand" means in accordance with the present invention an agent capable of inhibiting and/or modulating the interaction of TIRC7 with its corresponding ligand. Since the interaction of TIRC7 with its ligand(s) modulates events which are valuable in course of immune responses, such inhibitor should also be capable of modulating immune responses. In accordance with the present invention, said inhibitor preferably interacts with the TIRC7-ligand, for example by specifically binding to said ligand. "Specifically binding" means "specifically interacting with" whereby said interaction may be, inter alia, covalen non-covalent and/or hydrophobic. Such inhibitors are defined herein below or may be obtained by methods described herein. Potential inhibitors include small molecules which bind to, interfere with and/or occupy relevant sites on said ligand. Examples of small molecules include small peptides or peptide-like molecules.

The term "immune unresponsiveness" comprises non-unresponsiveness of immune cell subsets like T-cell or B-cells, NK-cells, monocytes and/or macrophages. Immune unresponsiveness can be maintained by blocking the stimulation of each ligand protein which binds to TIRC7 in a subject who has an autoimmune disease to alleviate symptoms of the autoimmune disease. In these cases, a TIRC7 or its ligand inhibhory agent is administered to the subject in an amount and over a period of time sufficient to maintain immune unresponsiveness. Alternatively, immune unresponsiveness can be reversed in a subject bearing a tumor to stimulate a tumor specific immune response or in a subject receiving a vaccine to enhance the efficacy of the vaccine. For example, a cell (e.g. a tumor cell) can be modified to express a TIRC7 ligand or a TIRC7 stimulatory agent can be administered to the subject bearing a tumor or who has had a tumor surgically removed to prevent recurrence of the tumor. Additionally, antigen-specific responsiveness can be restored to anergized immune cells in vitro by stimulating the immune cells through TIRC7 or its ligands. Responsive cells generated in vitro can then be administered to a subject.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e. arresting its development; or (c) relieving the disease, i.e. causing regression of the disease.

Furthermore, the term "subject" as employed herein relates to animals in need of amelioration, treatment and/or prevention of immunological diseases as disclosed herein. Most preferably said subject is a human.

The ligand is HLA-(Human Leukocyte associated Antigen) class II alpha (α) chain, also referred to as HLA-DR α-chain. These terms are used interchangeably herein.

HLA class II is a heterodimer of two transmembrane glycoproteins, the alpha and beta chains. Oriented with their amino terminal ends on the outside of the cell, both chains comprise two extracellular domains, each of 90-100 amino acids, connected to a short cytoplasmic tail by a hydrophobic amino acid sequence that makes a single pass through the cell membrane. In the alpha chain the membrane distal domain is known as alpha 1 and the membrane proximal domain as alpha 2. Likewise, in the beta chain the membrane distal domain is known as beta 1 and the membrane proximal domain as beta 2. Both membrane proximal domains possess structural characteristics of C1-type immune globulin domains. The alpha 1 and beta 1 domains are polymorph and occupied with presentations of peptides (12 - 24mers) to the T-cell receptor during the course of T-cell activation. Studies using site-specific mutants have mapped the site of CD4 binding to the membrane proximal beta 2 domain of the HLA class 11 molecule.
Expression of certain HLA class II molecules and their polymorphism are strongly associated with a number of diseases such as insulin-dependent diabetes mellitus, Goodpasture syndrome, Pemphigus vulgaris, Systemic lupus erythematosus, Multiple sclerosis, Grave's disease, Rheumatoid arthritis and Myastenia gravis.
Many of the diseases associated with HLA polymorphisms do not involve active infections and their symptoms are caused by a chronic state of inflammation and/or autoimmunity. In the case of diabetes, one general mechanism which has been proposed is that T-cells activated by presentation of a microbial antigen subsequently crossreact with self peptides to which they were tolerant before activation. Thus, Coxsackie virus infections have been correlated with diabetes and various bacterial infections of the intestine with the HLA class II associated diseases.
In infectious diseases relatively little is known of the effects of HLA polymorphism. From studies on cohorts of AIDS patients HLA effect can be seen, such as quicker progression of the disease (Roger et al., Faseb, 12, 1998, p. 625-32, Marsh, Parham and Barber, "The HLA-FACS-Book", Academic Press (2000), 37-97).

During the development the human immune response becomes tolerant of the normal components of healthy cells and tissues to which lymphocytes are exposed. It is of particular importance that a person's immune system develops tolerance to the self HLA class I and II allotypes expressed on the surface of that same person's cell. By contrast, a person's immune system is not tolerant of the many hundreds of non-self HLA allotypes expressed by other human beings such as after organ transplantation. Therefore, once a person receives a transplant, hyperacute or acute rejection of the transplanted organ is likely to occur if the recipient and donor are not compatible in their HLA antigen types expressed on the cell surface.

In context of the present invention, it was surprisingly found that the HLA-class 11 α chain is capable of interacting with TIRC7 as shown in the appended examples. Therefore, and without being bound by theory, it is envisaged that interfering with said binding/interaction between TIRC7 and the HLA-class 11 α chain leads to modifications of cellular responses due to the corresponding receptor/ligand interaction. Said modifications comprise, but are not limited to, inhibitory as well as stimulatory cellular responses. Preferably, however, irrespective of the theory behind the molecular mechanism of action, an inhibitor for use in accordance with the present invention can be characterized by (1) interacting/binding to TIRC7 or its ligand, and (2) being capable of inhibiting proliferation of mitogen-stimulated PBMCs in an assay as described In WO99/11782 and in Utku et al., Immunity 9 (1998), 509-518. Preferably, said inhibitor interacts/binds to said ligand of TIRC7 or to TIRC7 such that said inhibitor prevents said ligand from interacting with TIRC7, for example by occupying the site of TIRC7 with which said ligand interacts. As mentioned before, said ligand is the HLA-class II α chain. Nucleotide and amino acid sequences of human MHC class II HLA-DR-alpha chain are known to the person skilled in the art and can be retrieved from public databases, e.g., Genbank accession numbers V00523 and M60334. The nucleotide and amino acid sequences of the HLA-class II a chain that have been identified in accordance with the present invention are depicted in SEQ ID NO: -1 and SEQ ID NO: 2, respectively.

Accordingly, in a preferred embodiment of the present invention, said inhibitor is selected from the group consisting of:
(a) an agent binding to and/or interfering with a (poly)peptide or (a) fragment(s) thereof comprising an amino acid sequence as depicted in SEQ ID NO:
(b) an agent binding to a (poly)peptide or (a) fragment(s) thereof encoded by a polynucleotide comprising a nucleotide sequence as depicted in SEQ ID NO: 1; and
(c) an analogue or derivative of the (poly)peptide or the fragment(s) thereof as defined in (a) or (b);

Class II Major histocompatibility antigens (MHC) are composed of two noncovalently associated polypeptide chains, the beta2, alpha2 chains which contain internal disulfide bonds and judged by their amino acid sequence, belong to the ig superfamily and the atphal, beta1 chain which is the peptide binding domain of MHC class 11 molecules. The expression of MHC molecules on different cell types determines whether or not T-lymphocytes can interact with foreign antigens present On the surface of these cells MHC class II molecules are expressed on most-immune and non-immune human cells. The expression of MHC gene products is highly regulated at the level of transcription both by cell type specific factors and by inflammatory and immune stimuli, including cytokines like IFN-gamma. Some cells such as macrophages can be induced to express class II molecules by cytokines, especially IFN-gamma whereas other cells and B lymphocytes constituuvely express class II molecules. The activation of CD4 helper T-cells by antigen requires the participation of cells other than T-lymphocytes; these cells are often called accessory cells which express MHC class 11 molecules on their surface. The obligatory role of accessory cells in lymphocyte activation follows in two ways:
First, accessory cells are antigen presenting cells (APC) and they convert protein antigens to peptides and they present peptide-MHC-complexes In a form that can be recognized by CD4+ T-cells. The conversion of native proteins to MHC-associated peptide fragments by APCs is called antigen processing. The second function of accessory cells is to provide stimuli to the T-cell, beyond those initiated by peptide-MHC complexes binding to the T-cell antigen receptor. These stimuli, referred to as costimuiatonactivities, are required for full physiologic activation of the T-cells which are provided by membrane-bound or secreted products of accessory cells.

Immune response is strongly dependent on successful presentation of antigens to T-cells by MHC class I or II molecules on APCs. In case of insufficient antigen presentation to CD4 cells, the creation of immune response will not occur. Instead, unresponsiveness or ignorance will take place. This will lead to significant decrease of the ability of the immune system to destroy foreign particles required in such as infectious diseases as well as wound healing and sepsis. As demonstrated by Utku et al., Immunity 9 (1998), 509-518, targeting of TIRC7 by specific antibodies inhibits immune response and decreases TH-1 type lymphocyte cytokine expression such as IFN-gamma

In context of the present invention, it was surprisingly found that the polypeptide as depicted in SEQ ID NO: 2 is a protein capable of interacting with TIRC7, the cDNA of which has been isolated by screening of a yeast two hybrid library from human lymphocytes against TIRC7 polypeptide. As mentioned before, SEQ ID NO: 2 and its encoding nucleotide sequence as depicted in SEQ ID NO: 1 relate to the HLA-DR α-chain. HLA-DR binds to/interacts with TIRC7 as shown in the appended Examples. Therefore, and without being bound by theory, it is envisaged that said binding/interaction leads to modifications of cellular responses to the corresponding receptor/ligand interaction. Said modifications comprise, but are not limited to, inhibitory as well as stimulatory cellular responses.

The term "agent" as employed herein above relates to molecules like (poly)peptides, anorganic and/or organic substances as well as synthetically synthesized molecules. For example, said "agent" may comprise "small molecules", like peptides, anorganic and/or organic substances or peptide-like molecules, like peptide-analogues comprising D-amino acids. Said "agent" may also comprise nucleic acid molecules, like specific (oligo)nucleotides, PNAS and/or aptamers. In accordance with the present invention the term "agent" as employed herein also comprises substances which may be single substances or a plurality of substances which may or may not be identical. Said agent/compound may be comprised, for example, in all extracts from plants, animals or microorganisms.

The above mentioned polypeptide as set forth in SEQ ID NO: 2 is derived from a cDNA-clone "cDNA-screen7" as illustrated in the appended Examples. In accordance with the present invention the above recited polypeptide, corresponding to HLAII-DR comprises not only the precise amino acid sequence as set forth in SEQ ID NO: 2 but also comprises polypeptides which comprise amino acid sequences which show homology to said amino acid sequence and are capable of functioning as the HLAII-DR molecule ("cDNA-screen7" gene product) as disclosed herein. These homologous amino acid sequences may comprise, inter alia, "variants" of HLAII-DR. The term "variant" comprises but is not limited to allelic variants, splice variants, synthetically produced variants or genetically engineered variants. These variants can vary at either the polynucleotide and/or the polypeptide level. Alternatively, non-naturally occurring variants are also comprised as binding partner of the above identified agent These non-naturally occurring variants may, inter alia, be produced by mutagenesis techniques or by direct synthesis. Therefore, (an) agent(s) binding to and/or interfering with the herein above defined polypeptide also refer to agents which are capable of interacting with, binding to and/or interfering with such variants. Accordingly the term " fragment(s)" as used herein above refers to peptides and polypeptides that are derived from said HLAII-DR-molecule and that are capable of being bound and/or interacting with the above mentioned agent

The term "analogue or derivative" of the polypeptide or fragment thereof as used herein above denotes analogues or derivatives of the amino acid sequence as shown in SEQ ID NO: 2 and/or of HLAII-DR which are in contrast to the above defined variants of said molecule capable of specifically inhibiting the interaction of HLAII-DR and TIRC7. These analogues/derivatives, therefore, comprise, inter alia, chemically and/or genetically modified HLAII-DR-molecules which are not capable of eliciting physiological and/or cellular responses normally mediated by HLAII-DR - TIRC7 interactions. These analogues and/or derivatives may also, in accordance with the invention, be fusion proteins and/or mosaic polypeptides which comprise HLAII-DR and/or fragments thereof in combination with further molecules or polypeptide-fragments capable of interfering with the above recited HLAII-DR - TIRC7 interaction. The person skilled in the art is fully aware of methods/techniques for the preparation of such analogues/derivatives, fusion proteins and/or mosaic polypeptides; see, inter alia, Sambrook et al. (Molecular cloning; A Laboratory Manual. Second Edition, Cold Spring Harbor Laboratory Press; Cold Spring Harbor NY (1989)) or Oxender and Fox (1987) "Protein engineering", Liss New York. These techniques comprise, e.g., mutagenesis techniques, direct biochemical and/or chemical synthesis. Accordingly, said analogues/derivatives may be of natural origin or synthetic or semi-synthetic molecules.

In a further preferred embodiment of the present invention, said inhibitor is an aptamer or antibody specifically binding to the HLA-II α chain. It is envisaged that blockade of binding of TIRC7 by using anti-HLA-DR-antibodies leads to blockade of positive signals leading to inhibition of proliferation and is of great importance to modulate undesired immune reactions.

The term "aptamer" means nucleic acid molecules that can bind to target molecules, in accordance with this invention, i.e. the target molecule HLAII-DR as defined herein above. Aptamers commonly comprise RNA, ssDNA, modifie of RNA/DNA. The preparation of aptamers is known in the art and may involve, e.g., the use of combinatorial libraries to identify binding sites (Gold, Ann. Rev. Biochem. 64 (1995), 736-797).

As mentioned herein above, inhibitors capable of interfering with the interaction of TIRC7 with its ligand(s) may also comprise antibodies specifically binding to HLAII-DR-molecules as defined herein. Said antibodies may comprise monoclonal, polyclonal, synthetic, chimeric, humanized, xenogeneic and/or semi-synthetic antibodies. The term "antibody" also comprises fragments such as Fab, Fv-or scFv-fragments. Antibodies, fragments thereof, derivatives may be produced and/or obtained by known methods as described for example In Hariow and Lane "Antibodies, a Laboratory manual" CSH Press, 1988. The preparation of chimeric antibodies is described in WO89/09622.

Furthermore, the present invention relates to the use of a nucleic acid molecule encoding said inhibitor for the preparation of a pharmaceutical composition for the treatment of an immunological disorder, like graft versus host disease, autoimmune diseases, allergic diseases, infectious diseases, sepsis, for the treatment of tumors, for the improvement of wound healing or for inducing or maintaining immune unresponsiveness in a subject. Nucleic acid molecules as employed in the present invention comprise RNA, like mRNA, DNA, like cDNA. The term also comprises PNAs. In addition, the present invention relates to the use of a vector comprising the nucleic acid molecule as described herein above for the preparation of a pharmaceutical composition for the treatment of the above defined immunological disorders or for the improvement of wound healing or for inducing or maintaining immune unresponsiveness in a subject. The vector of the present invention may be a plasmid, cosmid, virus or other vector used, e.g. in gene therapeutic approaches. For further details see, for example, Molecular Cloning: a Laboratory Manual: 2nd edition, Sambrook et al, 1989, Cold Spring Harbor Laboratory Press. Many known techniques and protocols for manipulation of nucleic acid, for example in preparation of nucleic acid constructs, mutagenesis (see above), sequencing, introduction of DNA into cells and gene expression, and analysis of proteins, are described in detail in Current Protocols in Molecular Biology, Second Edition, Ausubel et al. eds., John Wley & Sons, 1994 and updated issues.

Furthermore, the present invention relates to a method of identifying and obtaining an agent capable of interfering with the interaction between TIRC7 and its ligand comprising the steps of:
(a) testing a collection of (poly)peptides or substances for their ability to inhibit interaction between TIRC7 and HLAII-DR using a suitable readout system; and
(b) identifying (poly)peptides or substances which test positive for inhibition of the interaction of TIRC7 with its ligand in step (a).
The above mentioned test of step (a) may, inter alia, comprise the testing of said (poly)peptides or substances (e.g. agents as defined herein above) in assays which allow the detection of binding to HLAII-DR-molecules. These (poly)peptides or substances may, by specifically binding to HLAII-DR-molecules, be capable of interfering with the TIRC7 - HLAII-DR signaling pathway. Said "test for ability to interfere/inhibit" the interaction between TIRC7 and its ligand, may, therefore, be carried out by specific immunological and/or biochemical assays known In the art Such assays comprise the measurement of complex formation, assays wherein binding partners are detected, like ELISAs, RIAs and the like. Said interaction assays may also comprise read-out systems known in the art iikep two-hybrid systems wherein, inter alia, binding partners of HLAII-DR can be detected (which may be used to interfere with the HLAII-DRlTIRC7 interaction) or in vitro binding assays. Said assays and corresponding read-out systems may also comprise the detection of complex formation and/or inhibition of such complex formations. For example, it can be tested, whether an in vitro complex of TIRC7/HLAII-DR (or fragments thereof) forms in presence of the (poly)peptide, substance, agent to be detected. Read-out systems in this context may be, inter alia, fluorescence kits and immunoassays, like immunoprecipitation assays. It is also envisaged that high throughput screening be employed in the methods of the present invention.

Hence, in one embodiment, the invention relates to a method for identifying and isolating an agent for treatment of immune diseases comprising the steps of:
(a) screening a host cell comprising a reporter gene whose transcription is directly or indirectly activated by dimers comprising TIRC7 and HLA-dass II α chain or their corresponding interacting binding domains with a compound to be screened; and
(b) selecting a compound that represses activation of the reporter gene

Thus, compounds that prevent the formation of dimers or multimeric complexes comprising TIRC7 and HLA-class II α chain can be used as a therapeutic intervention in TIRC7 associated diseases. Accordingly, this embodiment of the invention provides a method for the discovery of compounds that inhibit TIRC7 mediated and HLA-class II α chain associated signal transduction in responses of the immune system. The basic set up for assays in accordance with the method of the present invention is well known to the person skilled. This can be achieved by assays well known in the art, for example, as described in Scofield (Science 274 (1996), 2063-2065) by use of the so-called yeast "two-hybrid system". In this system the protein encoded by the TIRC7 encoding nucleic acid molecules or a smaller part thereof can be linked to the DNA-binding domain of the GAL4 transcription factor. A yeast strain expressing this fusion protein and comprising a lacZ reporter gene driven by an appropriate promoter, which is recognized by the GAL4 transcription factor, is transformed with a vector which expresses the protein ligand of TIRC7 or peptides thereof fused to an activation domain. Thus, in the absence of substances which interfere with the formation of dimers or multimeric complexes comprising TIRC7 and said ligand, e.g. HLA-class II α chain, the complex is able to direct expression of the reporter gene. For example, the two-hybrid assay system described in the Examples can be adapted accordingly. Thus, it is no longer used to screen an interaction partner of TIRC7 but for identification of substances that interfere with the interaction of TIRC7 with the already identified binding partner of TIRC7, i.e. the HI.A-class 11 a chain. Thus, in a preferred embodiment of the above described method, the host cell used in the screening assay comprises a. least one expression vector containing a DNA molecule encoding at least the HLA-class 11 a chain or a fragment thereof operatively linked to a transactivation or DNA binding domain. A. similar strategy can be pursued with the so called three hybrid system.

Other methods for identifying compounds which interfere with formation of dimers or multimeric complexes comprising TIRC7 and said ligand, e.g. HLA-class 11 α chain are, for example, the in vitro screening with the phage display system as well as filter binding assays or "real time" measuring of interaction using, for example, the BlAcore apparatus (Pharmacia); see references cited supra. '

in a preferred embodiment, the present invention relates to a method of Identifying an agent capable of interfering with the interaction between TIRC7 and its ligand, further comprising the step of
(c) repeating steps (a) and (b) with the (poly)peptides or substances identified one or more times wherein the newly identified (poly)peptide or substance replaces the previously identified (poly)peptide or substance as a bait for the identification of a further interacting (poly)peptide or substance.

Additionally, the present invention relates to a method of identifying and obtaining a (poly)peptide involved in the regulation of the immune response in a subject comprising the steps of :
(a) contacting a collection of (poly)peptides with (a) (poly)peptide(s) or fragment(s) thereof having an amino acid sequence as depicted in SEQ ID NO: 2 or being encoded by SEQ ID NO: 1 or obtainable by the methods as disclosed herein, under suitable conditions that allow binding of said (poly)peptides;
(b) removing (poly)peptides from said collection of (poly)peptides that did not bind to said (poly)peptide(s) or fragment(s) thereof as defined in (a); and
(c) identifying (poly)peptides that bind to said (poly)peptide(s) or fragment(s) thereof as defined in (a).

The compounds and collection of (poly)peptides which can be tested and identified according to a method of the invention may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). In a preferred embodiment of any one of the above described methods of the invention, cell lysates are used derived from, for example, tumor tissue or cells of the immune system, most preferably a human lymphocyte lysate is used; see the Examples and Figure 3.

Furthermore, the present invention relates to a method of refining an inhibitor as defined herein or an agent or a (poly)peptide identified by the methods as disclosed herein comprising :
(a) modeling said agent or (poly)peptide by peptidomimetics; and
(b) chemically synthesizing the modeled compound.
Thus, the above-described methods can, of course, be combined with one or more steps of any of the above-described screening methods or other screening methods well known in the art. Methods for clinical compound discovery comprise for example ultrahigh-throughput screening (Sundberg, Curr. Opin. Blotechnol. 11 (2000), 47-53) for lead identification, and structure-based drug design (Veninde and Hol, Structure 2 (1994), 577-587) and combinatorial chemistry (Salemme et al., Structure 15 (1997), 319-324) for lead optimization. Once a drug has been selected, the method can have the additional step of repeating the method used to perform rational drug design using the modified drug and to assess whether said modified drug displays better affinity according to for example interaction/energy analysis.
Accordingly, the compounds isolated by the above methods can also serve as lead compounds for the development of analog compounds. The analogs should have a stabilized electronic configuration and molecular conformation that allows key functional groups to be presented to TIRC7 or its ligand in substantially the same way as the lead compound. In particular, the analog compounds have spatial electronic properties which are comparable to the binding region, but can be smaller molecules than the lead compound, frequently having a molecular weight below about 2 kD and preferably below about 1 kD. Identification of analog compounds can be performed through use of techniques such as self consistent field (SCF) analysis, configuration interaction (Cl) analysis, and normal mode dynamics analysis. Computer programs for implementing these techniques are available; e.g., Rein, Computer-Assisted Modeling of Receptor-Ligand Interactions (Alan Liss, New York, 1989). Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art; see also supra. Furthermore, peptidomimetics and/or computer aided design of appropriate derivatives and analogues can be used, for example, according to the methods described above. Methods for the lead generation in drug discovery also include using proteins and detection methods such as mass spectrometry (Cheng et al. J. Am. Chem. Soc. 117 (1995), 8859-8860) and some nuclear magnetic resonance (NMR) methods (Fejzo et al., Chem. Biol. 6 (1999), 755-769; Lin et al., J. Org. Chem. 62 (1997), 8930-8931).

The inhibitor, agents and (poly)peptides used, in the compositions of the present invention preferably have a specificity at least substantially identical to the binding specificity of the TIRC7 with its ligand, said ligand being HLA-class II α chain, in particular if TIRC7 stimulation is desired. An inhibitor can have a binding affinity to the HLA-class II α chain protein of at least 10⁵ M⁻¹, preferably higher than 10⁷ M⁻¹ and advantageously up to 10¹⁰ M⁻¹ in case TIRC7 mediated proliferation of immune cells should be suppressed. In a preferred embodiment, a suppressive agent or inhibitor has an affinity of at least about 10⁻⁷ M, preferably at least about 10⁻⁸M and most preferably at least about 10⁻¹¹ M. In case of antisense nucleic acid molecules it is preferred that they have a binding affinity to those encoding the HLA-class II α chain of at most 2-, 5- or 10-fold less than an exact complement of 20 consecutive nucleotides of the coding sequence.

Preferably, the inhibitor, agent or (poly)peptide is not larger than the "bioavailability wall" of 500-600 Da in order to be able to cross the lipophilic cell membrane into the cell. On the other hand, in protein therapy it has been recently demonstrated that enzymes fused to part of a protein from the HIV virus can cross cell membranes while retaining their enzymatic activity in vivo in mice (Schwarze, Science 285 (1999), 1569-1572). It has been known for approximately ten years that the transactivating regulatory protein (TAT protein) from the HI virus has an unusual ability to cross cell membranes without using receptors or transporters, or requiring ATP (Green and Loewenstein, Cell 55 (1988), 1179-1188). Although its exact mechanism is unknown, it has been shown that the protein transduction domain (PTD) of TAT opens a "hole" in the cell membrane lipid bilayer, pulling anything covalently attached through it, before closing it again. This is a specific process that does not otherwise damage the cell. Thus, a functional inhibitor agent or (poly)peptide identified by a method of the present invention may be coupled to PTD via a linker in order to let them cross the cell membrane; see also for review DDT 4 (1999), 537.

In addition, the present invention relates to a method of producing a composition comprising formulating the inhibitor as disclosed herein, an agent identified by the methods as disclosed herein, the (poly)peptide identified by the method as disclosed herein or an agent or (poly)peptide refined by the method as disclosed herein and, optionally, a pharmaceutically acceptable carrier and/or diluent

Once a drug has been selected in accordance with any one of the above-described methods of the present invention, the drug or.a pro-drug thereof can be synthesized in a therapeutically effective amount. As used herein, the term "therapeutically effective amount" means the total amount of the drug or pro-drug that is sufficient to show a meaningful patient benefit, i.e., treatment, healing, prevention or amelioration of, for example, an immune disease, or an increase in rate of treatment, healing, prevention or amelioration of such conditions. In addition or alternatively, in particular with respect to pre-clinical testing of the drug the term "therapeutically effective amount" includes the total amount of the drug or pro-drug that is sufficient to elicit a physiological response, preferably upon its binding to its target TIRC7 or its ligand, in an non-human animal test.

Drugs or pro-drugs after their *in vivo* administration are metabolized in order to be eliminated either by excretion or by metabolism to one or more active or Inactive metabolites (Meyer, J. Pharmacokinet Biopharm. 24 (1996), 449-459). Thus, rather than using the actual compound or drug identified and obtained in accordance with the methods of the present invention a corresponding formulation as a pro-drug can be used which is converted into its active form in the patient Precautionary measures that may be taken for the application of pro-drugs and drugs are described in the literature; see, for review, Ozama, J. Toxicol. Sci. 21 (1996), 323-329.

ln.a preferred embodiment, the present invention relates to the method as disdosed herein, wherein the composition is a. pharmaceutical composition. For this embodiment any one of the above described methods may comprise the steps of (a) modifying an agent identified by the method of the invention as a lead compound to achieve (i) modified site of action, spectrum of activity, organ specificity, and/or (il) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterfication of hydroxyl groups with carbon acids, or (iii) esterfication of hydroxyl groups to, e.g. phosphates, pyrophosphates or sulfates or hemi succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiffs bases, oximes, acetales, ketales, enolesters, oxazolidines, thiozolidines or combinations thereof; and (b) formulating the product of said modification with a pharmaceutically acceptable carrier.
The various steps recited above are generally known in the art. They include or rely on quantitative structure-action relationship. (QSAR) analyses (Kubinyi, I. Med. Chem. 41 (1998), 22553-2564; Pharm.. Unserer Zeit 23 (1994), 281-290), combinatorial biochemistry, classical chemistry and others.

The term "pharmaceutical composition", in context of this invention, optionally, further comprises other molecules, either alone or in combination, like e.g. molecules which are capable of modulating and/or interfering with the immune system. The pharmaceutical composition may be in solid, liquid or gaseous form and may be, inter alia, in a form of (a) powder(s), (a) tablet(s), (a) solution(s) or (an) aerosol(s).
The pharmaceutical composition of the present invention may further comprise a pharmaceutically acceptable carrier. Examples of suitable pharmaceutical carriers are well known in the art and include phosphate buffered saline solutions, water, emulsions, such as oivwater emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical or intradermal administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently.

Furthermore, the present invention relates to the use of an inhibitor as disclosed herein, an agent or a (poly)peptide as identified by the methods as disclosed herein or of an inhibitor as refined by the method as disclosed herein for the preparation of a pharmaceutical composition for the treatment of graft versus host disease, autoimmune diseases, allergic diseases, infectious diseases, sepsis, for the treatment of tumors, for the improvement of wound healing or for inducing or maintaining immune unresponsiveness in a subject.

Additionally, the present invention relates to a pharmaceutical composition comprising an inhibitor, a nucleic acid molecule, a vector and/or an agent as defined herein and/or a (poly)peptide identified by the method disclosed herein.

Furthermore, the present invention relates to a non-human transgenic animal overexpressing a gene product encoded by a nucleic acid sequence comprising a nucleotide sequence as defined herein above. Furthermore, the present invention also provides for a non-human transgenic animal, wherein the nucleic acid molecule as defined herein above or a homolog, para log or ortholog thereof is silenced and/or mutated. Said non-human transgenic animals are particularly useful for medical and scientific research purposes and may comprise mice, rats, dogs, sheep and the like as well as non-vertebrates, like C. etegans or Drosophila. The non-human animal can be used in accordance with a screening method of the invention described herein. Production of transgenic embryos and screening of those can be performed, e.g., as described by A. L Joyner Ed., .Gene Targeting, A Practical Approach (1993), Oxford University Press. The DNA of the embryonal membranes of embryos can be analyzed using, e.g., Southern blots with an appropriate probe.

Furthermore, the present invention relates to the use of the HLA-class II α chain or a fragment or derivative for the identification of drugs for the treatment of an immune disease or tumor or as a ligand in any one of the above defined methods.

In a further aspect, the present invention relates to a method of diagnosing a pathological condition or a susceptibility to a pathological condition in a subject related to a disorder which is mediated by or responsive to the activity of TIRC7, comprising:
(a) determining the presence or absence of a mutation in a polynucleotide encoding HLA-class II α chain; and
(b) diagnosing a pathological condition or a susceptibility to a pathological condition based on the presence or absence of said mutation.
In addition, the present invention relates to a method of diagnosing a pathological condition or a susceptibility to a pathological condition in a subject related to a disorder which is mediated by or responsive to the activity of TIRC7, comprising:
(a) determining the presence or amount of expression of a HLA-class II α chain polypeptide in a biological sample; and
(b) diagnosing a pathological condition or a susceptibility to a pathological condition based on the presence or amount of expression of the polypeptide.
In these embodiments, the HLA-class II a chain polynucleotides, nucleic acid molecules, (poly)peptide, antibodies or compounds identified above are preferably detectably labeled. A variety of techniques are available for labeling biomolecules, are well known to the person skilled in the art and are considered to be within the scope of the present invention. Such techniques are, e.g., described in Tijssen, "Practice and theory of enzyme immuno assays". Burden, RH arid von. Knippenburg (Eds), Volume 15 (1985), "Basic methods in molecular biology"; Davis LG, Dibmer MD; Battey Elsevier (1990), Mayer et al., (Eds) "Immunochemical methods in cell and molecular biology" Academic Press, London (1987), or in the series "Methods in Enzymology", Academic Press, Inc. There are many different labels and methods of labeling known to those of ordinary skill in the art. Commohly used labels comprise, inter alia, fluorochromes (like fluorescein, rhodamine, Texas Red, etc.), enzymes (like horse radish peroxidase, β-galactosldase, alkaline phosphatase), radioactive isotopes (like ³²P or ¹²⁵I), biotin, digoxygenin, colloidal metals, chemo- or bioluminescent compounds (like dioxetanes, luminol or acridiniums). Labeling procedures, like covalent coupling of enzymes or biotinyl groups, lodinations, phosphorylations, biotinylations, random priming, nick-translations, tailing (using terminal transferases) are well known in the art. Detection methods comprise, but are not limited to, autoradiography, fluorescence microscopy, direct and indirect enzymatic reactions, etc.
In addition, the above-described compounds etc. may be attached to a solid phase. Solid phases are known to those skilled in the art and may comprise polystyrene beads, latex beads, magnetic beads, colloid metal particles, glass and/or silicon chips and surfaces, nitrocellulose strips, membranes, sheets, animal red blood cells, or red blood cell ghosts, duracytes and the walls of wells of a reaction tray, plastic tubes or other test tubes. Suitable methods of immobilizing HLA-class II α chain nucleic acids, (poly)peptides, proteins, antibodies, etc. on solid phases include but are not limited to ionic, hydrophobic, covalent interactions and the like. The solid phase can retain one or more additional receptor(s) which has/have the ability to attract and immobilize the region as defined above. This receptor can comprise a charged substance that is oppositely charged with respect to the reagent itsetf or to a charged substance conjugated to the capture reagent or the receptor can be any specific binding partner which is immobilized upon (attached to) the solid phase and which is able to immobilize the reagent as defined above.
Commonly used detection assays can comprise radioisotopic or non-radioisotopic methods. These comprise, inter alia, RIA (Radioisotopic Assay) and IRMA (immune Radioimmunometric Assay), EIA (Enzym lmmuno Assay), ELISA (Enzyme Linked immune Assay), FIA (Fluorescent immune Assay),' and CLIA (Chemoluminescent Immune Assay). Other detection methods that are used in the art are those that do not utilize tracer molecules. One prototype of these methods is the agglutination assay, based on the property of a given molecule to bridge at least two particles.

For diagnosis and quantification of (poly)peptides, polynucleotides, etc. in clinical and/or scientific specimens, a variety of immunological methods, as described above as well as molecular biological methods, like nucleic acid hybridization assays, PCR assays or DNA Enzyme Immunoassays (Mantero et al., Clinical Chemistry 37 (1991), 422-429) have been developed and are well known in the art. In this context, it should be noted that the HLA-class II α chain nucleic acid molecules may also comprise PNAs, modified DNA analogs containing amide backbone linkages. Such PNAs are useful, inter alia, as probes for DNA/RNA hybridization.

The above-described compositions may be used for methods for detecting expression of a HLA-class II α chain polynucleotide by detecting the presence of mRNA coding for a HLA-class II α chain (poly)peptide which comprises, for example, obtaining mRNA from cells of a subject and contacting the mRNA so obtained with a probe/primer comprising a nucleic acid molecule capable of specifically hybridizing with a HLA-class II α chain polynucleotide under suitable hybridization conditions, and detecting the presence of mRNA hybridized to the probe/primer. Further diagnostic methods leading to the detection of nucleic acid molecules in a sample comprise, e.g., polymerase chain reaction (PCR), ligase chain reaction (LCR), Southern blotting in combination with nucleic acid hybridization, comparative genome hybridization (CGH) or representative difference analysis (RDA). These methods for assaying for the presence of nucleic acid molecules are known in the art and can be carried out without any undue experimentation.

Furthermore, the invention comprises methods of detecting the presence of a HLA-class 11 α chain protein in a sample, for example, a cell sample, which comprises obtaining a cell sample from a subject, contacting said sample with one of the aforementioned antibodies under conditions permitting binding of the antibody to the HLA-class II α chain protein, and detecting the presence of the antibody so bound, for example, using immuno assay techniques such as radioimmunoassay or enzymeimmunoassay. Furthermore, one skilled in the art may specifically detect and distinguish polypeptides which are functional HLA-class II α chain proteins from mutated forms which have lost or altered their HLA-class II α chain activity by using an antibody which either specifically recognizes a (poly)peptide which has HLA-class II a chain activity but does not recognize an inactive form thereof or which specifically recognizes an inactive form but not the corresponding polypeptide having HLA-class II α chain activity.

Furthermore, the present invention relates to a method as described above wherein said sample is or is derived from hair, blood, serum, sputum, feces or another body fluid. The sample to be analyzed may be treated such as to extract, inter alia, nucleic acid molecules, (poly)peptides, or antibodies.

Preferably, the condition or immune disease mediated by TIRC7 and to be diagnosed in accordance with the above described methods is one of the group consisting of graft versus host disease, autoimmune disease, allergic disease, infectious disease, sepsis, tumor, in particular those involving immune cells, deficiency in wound healing and deficiency of a subject to elicit appropriate immune responses.

The present invention also relates to uses of kit compositions containing HLA-class II α chain specific reagents such as those described herein-before. Kits containing HLA-class II α chain DNA or RNA, antibodies to HLA-class II α chain, or HLA-class II α chain protein may be prepared. Such kits are used to detect DNA which hybridizes to HLA-class 11 α chain DNA or RNA or to detect the presence of HLA-class If α chain protein or peptide fragments in a sample. Such characterization is useful for a variety of purposes including but not limited to forensic analyses, diagnostic applications, and epidemiological studies in accordance with the above-described methods of the present invention. The recombinant HLA-class II α chain proteins, DNA molecules, RNA molecules and antibodies lend themselves to the formulation of kits suitable for the detection and typing of HLA-class II α chain. Such a kit would typically comprise a compartmentalized carrier suitable to hold in close confinement at least one container. The carrier would further comprise reagents such as recombinant HLA-class If α chain protein or anti- HLA-class II α chain antibodies suitable for detecting HLA-class II α chain. The carrier may also contain a means for detection such as labeled antigen or enzyme substrates or the like.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, hftp:/twww.tigr.org/, are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

This disclosure may best be understood in conjunction with the accompanying drawings, incorporated herein by references. Furthermore, a better understanding of the present invention and of its many advantages will be had from the following examples, given by way of illustration and are not intended as limiting.

Unless stated otherwise in the examples, all recombinant DNA techniques are performed according to protocols as described in Sambrook et al. (1989), Molecular Cloning: A Laboratory Manual. Cold Spring Harbor Laboratory Press, NY or in Volumes 1 and 2 of Ausubel et al. (1994), Current Protocols in Molecular Biology, Current Protocols.

The figures show:

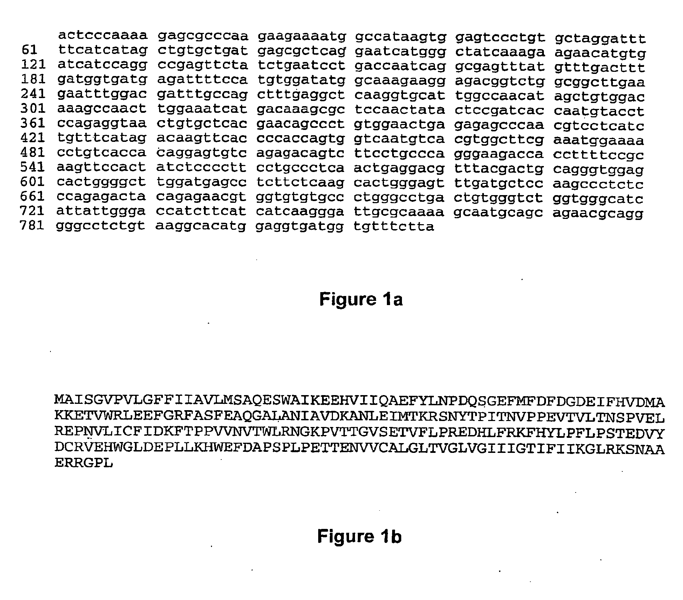

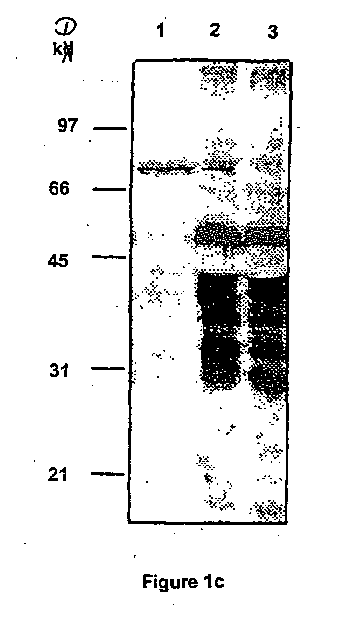

- Figure 1a:: Shown is the cDNA sequence of human HLA DR alpha chain which contains 819 nucleotides and demonstrates the cDNA which has been isolated by screening of a yeast two hybrid library from human lymphocytes.
- **Figure 1b:**: The amino acid sequence of HLA DR molecule is shown which includes 254 amino acids starting with a methionin.
- **Figure 1c**: Shown is the coimmunoprecipitation of TIRC7 protein with HLA DR protein from human lymphocyte lysate by using specific anti-TIRC7 and anti HLA DR alpha chain antibodies.

The Examples illustrate the invention.

### Example 1: Identification of a ligand of TIRC7 by using two hybrid library screening

The TIRC7 cDNA published by Utku et al., Immunity 9 (1998), 509-518, was used to perform two-hybrid screening using the HYBRZAP 2.1 system by Stratagene, La Jolla, USA. Positive clones were assayed following the instructions of the distributor. The clone "cDNA-screen7" revealed a cDNA insert of 819bp length which showed 100 % identity with human leukocyte antigen class II-DR (Figure 1a-1b).

### Example 2: Immunoprecipitation of HLA-DR alpha chain

Coimmunoprecipitation was performed as described in Jöns et al., Histochem. Cell. Biol., 1999, 111 (4), 313-8. Briefty, activated human peripheral blood lymphocyte lysate (Figure 1c, lane 1) was used for immunoprecipitation of HLA-DR alpha chain by utilizing the CBL120 monoclonal antibody (Cybus-Biotechnology) (Figure 1c, lane 2). In contrast, the control antibody, isotype IgG1 (Pharmingen) does not show any precipitation (Figure 1c, lane 3).

## Claims

1. Use of an inhibitor interfering with the interaction of TIRC7 with its ligand for the preparation of a pharmaceutical composition for the treatment of graft versus host disease, autoimmune diseases, allergic diseases, infectious diseases, sepsis, for the treatment of tumors, for the improvement of wound healing or for inducing or maintaining immune unresponsiveness in a subject, wherein said ligand is HLA-(Human Leukocyte associated Antigen) class II alpha (α) chain.

2. The use of claim 1, wherein said inhibitor is selected from the group consisting of:
(a) an agent binding to and/or interfering with a (poly)peptide or (a) fragment(s) thereof comprising an amino acid sequence as depicted in SEQ ID NO: 2;
(b) an agent binding to a (poly)peptide or (a) fragment(s) thereof encoded by a polynucleotide comprising a nucleotide sequence as depicted in SEQ ID NO: 1; and
(c) an analogue or derivative of the (poly)peptide or the fragment(s) thereof as defined in (a) or (b).

3. The use of claim 1 or 2, wherein said inhibitor is an aptamer or antibody specifically binding to the (poly)peptide(s) as defined in claim 1 or 2.

4. Use of a nucleic acid molecule encoding the inhibitor of any one of claims 1 to 3 for the preparation of a pharmaceutical composition for the treatment of graft versus host disease, autoimmune diseases, allergic diseases, infectious diseases, sepsis, for the treatment of tumors, for the improvement of wound healing or for inducing or maintaining immune unresponsiveness in a subject.

5. The use of claim 4, wherein said nucleic acid molecule is comprised in a vector.

6. A method of identifying and obtaining an agent capable of interfering with the interaction between TIRC7 and its ligand comprising the steps of :
(a) testing a collection of (poly)peptides or substances for their ability to inhibit interaction between TIRC7 and HLA-class II a chain (poly)peptides or (a) fragment(s) thereof using a suitable readout system; and
(b) identifying (poly)peptides or substances which test positive for inhibition of the interaction of TIRC7 with its ligand in step (a).

7. The method of claim 6, wherein said HLA-class II α chain (poly)peptides or (a) fragment(s) thereof comprise an amino acid sequence derived from SEQ ID NO: 2 or being encoded by SEQ ID NO: 1.

8. The method of claim 6 or 7, further comprising the step of ;
(c) repeating steps (a) and (b) with the (poly)peptides or substances identified one or more times, wherein the newly identified (poly)peptide or substance replaces the HLA-class II α chain (poly)peptides as a bait for the identification of a further interacting (poly)peptide or substance and is replaced in subsequent rounds by the interacting (poly)peptide or substance identified in the previous round.

9. The method of claim 8 for identifying and obtaining a (poly)peptide involved in the regulation of the immune response in a subject.

10. A method for identifying and isolating an agent for treatment of immune diseases comprising the steps of:
(a) screening a host cell comprising a reporter gene whose transcription is directly or indirectly activated by dimers comprising TIRC7 and HLA-class 11 a chain or their corresponding interacting binding domains with a compound to be screened; and
(b) selecting a compound that represses activation of the reporter gene.

11. The method of claim 10, wherein said host cell comprises at least one expression vector containing a DNA molecule encoding at least the HLA-class II α chain or a fragment thereof operatively linked to a transactivation or DNA binding domain.

12. The method of any one of claims 6 to 11 further comprising :
(a) modeling said agent or (poly)peptide by peptidomimetics; and
(b) chemically synthesizing the modeled compound.

13. Use of the HLA-class II α chain or a fragment or derivative thereof in a method of any one of claims 6 to 12.

14. An in vitro method of diagnosing a pathological condition or a susceptibility to a pathological condition in a subject related to a disorder which is mediated by or responsive to the activity of TIRC7, comprising:
(a) detennining the presence or absence of a mutation in a polynucleotide encoding the HLA-class II a chain; and
(b) diagnosing a pathological condition or a susceptibility to a pathological condition based on the presence or absence of said mutation,
wherein said disorder is selected from the group consisting of graft versus host disease, autoimmune diseases, allergic diseases, infectious diseases, sepsis, tumors, deficiency in wound healing and deficiency of a subject to elicit appropriate immune responses.

15. An in vitro method of diagnosing a pathological condition or a susceptibility to a pathological condition in a subject related to a disorder which is mediated by or responsive to the activity of TIRC7, comprising:
(a) determining the presence or amount of expression of HLA-class II α chain polypeptide in a biological sample; and
(b) diagnosing a pathological condition or a susceptibility to a pathological condition based on the presence or amount of expression of the polypeptide,
wherein said disorder is selected from the group consisting of graft versus host disease, autoimmune diseases, allergic diseases, infectious diseases, sepsis, tumors, deficiency in wound healing and deficiency of a subject to elicit appropriate immune responses.

16. Use of a kit in a method of any one of claims 6 to 15, said kit comprising a HLA-class II α chain polypeptide or a biologically active fragment thereof, a nucleic acid molecule encoding HLA-class II α chain or a nucleic acid molecule of at least 15 nucleotides in length hybridizing to a HLA-class II α chain gene, or an anti-HLA-class II α chain antibody, and optionally suitable means for detection.

## Patentansprüche

1. Verwendung eines Inhibitors, welcher in die Interaktion von TIRC7 mit seinem Liganden eingreift, zur Herstellung einer pharmazeutischen Zusammensetzung zur Behandlung von Graft-versus-Host-Reaktion, Autoimmunkrankheiten, allergischen Krankheiten, infektiösen Krankheiten, Sepsis, zur Behandlung von Tumoren, zur Verbesserung der Wundheilung oder zur Induzierung oder Aufrechterhaltung der Immununempfindlichkeit in einem Subjekt, wobei dieser Ligand HLA-(Human Leukocyte associated Antigen) Klasse II alpha-(α) Kette ist.

2. Verwendung nach Anspruch 1, wobei der Inhibitor ausgewählt ist aus der Gruppe bestehend aus:
(a) einem Agens, welches ein (Poly-) Peptid, umfassend eine Aminosäuresequenz wie in SEQ ID NO: 2 dargestellt, oder (ein) Fragment(e) davon bindet und/oder damit interferiert;
(b) einem Agens, welches an ein (Poly-) Peptid, kodiert durch ein Polynukleotid, umfassend eine Nukleotidsequenz wie in SEQ ID NO: 1 dargestellt, oder (ein) Fragment(e) davon bindet; und
(c) einem Analogon oder Derivat des (Poly-) Peptids oder des(der) Fragments(e) davon, wie in (a) oder (b) beschrieben.

3. Verwendung nach Anspruch 1 oder 2, wobei der Inhibitor ein Aptamer oder Antikörper ist, der spezifisch an das(die) (Poly-) Peptid(e), wie in Anspruch 1 oder 2 definiert, bindet.

4. Verwendung eines Nukleinsäuremoleküls, das den Inhibitor einer der Ansprüche 1 bis 3 kodiert, zur Herstellung einer pharmazeutischen Zusammensetzung zur Behandlung von Graft-versus-Host-Reaktion, Autoimmunkrankheiten, allergischen Krankheiten, infektiösen Krankheiten, Sepsis, zur Behandlung von Tumoren, zur Verbesserung der Wundheilung oder zur Induzierung oder Aufrechterhaltung der Immununempfindlichkeit in einem Subjekt.

5. Verwendung nach Anspruch 4, wobei das Nukleinsäuremolekül in einem Vektor enthalten ist.

6. Verfahren zur Identifizierung und Gewinnung eines Agens, welches in der Lage ist, in die Interaktion zwischen TIRC7 und seinem Liganden einzugreifen, umfassend die folgenden Schritte:
(a) Testen einer Kollektion von (Poly-) Peptiden oder Substanzen auf ihre Fähigkeit, eine Interaktion zwischen TIRC7 und HLA-Klasse II α-Ketten-(Poly-) Peptiden oder (einem) Fragment(en) davon zu inhibieren unter Verwendung eines geeigneten Auslesesystems; und
(b) Identifizierung von (Poly-) Peptiden oder Substanzen, welche in Schritt (a) positiv hinsichtlich der Inhibierung der Interaktion von TIRC7 mit seinem Liganden getestet wurden.

7. Verfahren nach Anspruch 6, wobei HLA-Klasse II a-Ketten-(Poly-) Peptide oder (ein) Fragment(e) davon eine Aminosäuresequenz umfassen, die von SEQ ID NO: 2 abgeleitet wurde oder durch SEQ ID NO: 1 kodiert wird.

8. Verfahren nach Anspruch 6 oder 7, des weiteren umfassend den Schritt:
(c) Ein- oder mehrmaliges Wiederholen der Schritte (a) und (b) mit den identifizierten (Poly-) Peptiden oder Substanzen, wobei das/die neu identifizierte (Poly-) Peptid oder Substanz die HLA-Klasse II α-Ketten-(Poly-) Peptide ersetzt und dabei als Köder für die Identifizierung eines/r weiteren interagierenden (Poly-) Peptids oder Substanz fungiert und in nachfolgenden Runden durch das/die interagierende (Poly-) Peptid oder Substanz, welche/s in der vorherigen Runde identifiziert wurde, ersetzt wird.

9. Verfahren nach Anspruch 8 zur Identifizierung und Gewinnung eines (Poly-) Peptids, das in die Regulierung der Immunantwort in einem Subjekt involviert ist.

10. Verfahren zur Identifizierung und Isolierung eines Agens zur Behandlung von Immunkrankheiten, umfassend die folgenden Schritte:
(a) Durchmustern einer Wirtszelle, umfassend ein Reportergen, dessen Transkription direkt oder indirekt aktiviert wird durch Dimere, umfassend TIRC7 und HLA-Klasse II α-Kette oder deren entsprechende interagierende Bindungsdomänen mit einer zu screenenden Verbindung; und
(b) Selektionierung einer Verbindung, welche die Aktivierung des Reportergens unterdrückt.

11. Verfahren nach Anspruch 10, wobei die Wirtszelle wenigstens einen Expressionsvektor umfasst, welcher ein DNA-Molekül enthält, welches zumindest die HLA-Klasse II α-Kette oder ein operativ mit einer Transaktivierungs- oder DNAbindenden Domäne verknüpftes Fragment davon kodiert.

12. Verfahren nach einem der Ansprüche 6 bis 11, des weiteren umfassend:
(a) Modellierung des Agens oder eines (Poly-) Peptids mittels Peptidomimetika; und
(b) chemische Synthese der modellierten Verbindung.

13. Verwendung der HLA-Klasse II α-Kette oder eines Fragments oder Derivats davon in einem Verfahren nach einem der Ansprüche 6 bis 12.

14. In vitro Verfahren zur Diagnose eines pathologischen Zustands oder einer Anfälligkeit für einen pathologischen Zustand in einem Subjekt, welche/r mit einer Störung zusammenhängt, die von der Aktivität von TIRC7 vermittelt wird oder für diese empfindlich ist, umfassend:
(a) Bestimmung der Anwesenheit oder Abwesenheit einer Mutation in einem Polynukleotid, welches für HLA-Klasse II α-Kette kodiert; und
(b) Diagnose eines pathologischen Zustands oder einer Anfälligkeit für einen pathologischen Zustand, welche/r auf der Anwesenheit oder Abwesenheit dieser Mutation basiert,
wobei diese Störung ausgewählt ist aus der Gruppe bestehend aus Graft-versus-Host-Reaktion, Autoimmunkrankheiten, allergischen Krankheiten, infektiösen Krankheiten, Sepsis, Tumoren, unzulängliche Wundheilung und die Unzulänglichkeit eines Subjekts, angemessene Immunantworten zu produzieren.

15. In vitro Verfahren zur Diagnose eines pathologischen Zustands oder einer Anfälligkeit für einen pathologischen Zustand in einem Subjekt, welche/r mit einer Störung zusammenhängt, die von der Aktivität von TIRC7 vermittelt wird oder für diese empfindlich ist, umfassend:
(a) Bestimmung der Anwesenheit oder der Menge der Expression eines HLA-Klasse II α-Ketten-Polypeptids in einer biologischen Probe; und
(b) Diagnose eines pathologischen Zustands oder einer Anfälligkeit für einen pathologischen Zustand, welche/r auf der Anwesenheit oder der Menge der Expression des Polypeptids basiert,
wobei diese Störung ausgewählt ist aus der Gruppe bestehend aus Graft-versus-Host-Reaktion, Autoimmunkrankheiten, allergischen Krankheiten, infektiösen Krankheiten, Sepsis, Tumoren, unzulängliche Wundheilung und Unzulänglichkeit eines Subjekts, angemessene Immunantworten zu produzieren.

16. Verwendung eines Kits in einem Verfahren nach einem der Ansprüche 6 bis 15, wobei das Kit ein HLA-Klasse II α-Ketten-Polypeptid oder ein biologisch aktives Fragment davon, ein Nukleinsäuremolekül, kodierend HLA-Klasse II α-Kette, oder ein Nukleinsäuremolekül von mindestens 15 Nukleotiden Länge, welches an ein HLA-Klasse II α-Ketten-Gen hybridisiert, oder einen anti-HLA-Klasse II α-Ketten-Antikörper, und gegebenenfalls geeignete Mittel zum Nachweis umfasst.

## Revendications

1. Utilisation d'un inhibiteur interférant avec l'interaction de la TIRC7 avec son ligand pour la préparation d'une composition pharmaceutique destinée au traitement de la maladie du greffon contre l'hôte, des maladies auto-immunes, des maladies allergiques, des maladies infectieuses, de la sepsie, au traitement des tumeurs, à l'amélioration de la cicatrisation et à l'induction ou au maintien d'une insensibilité immunitaire chez un sujet, dans laquelle ledit ligand est la chaîne alpha ( ) du HLA (antigène associé aux leucocytes humains) de classe II.

2. Utilisation selon la revendication 1, dans laquelle ledit inhibiteur est choisi dans le groupe constitué par :
(a) un agent se liant à et/ou interférant avec un (poly)peptide ou (a) un ou des fragments de celui-ci comprenant une séquence d'acides aminés telle que représentée par SEQ ID N° : 2 ;
(b) un agent se liant à un (poly)peptide ou (a) un ou des fragments de celui-ci codés par un polynucléotide comprenant une séquence nucléotidique telle que représentée par SEQ ID N° : 1 ; et
(c) un analogue ou un dérivé du (poly)peptide ou du ou des fragments de celui-ci tels que définis en (a) ou (b).

3. Utilisation selon la revendication 1 ou 2, dans laquelle ledit inhibiteur est un aptamère ou un anticorps se liant spécifiquement au(x) (poly)peptide(s) tel(s) que défini(s) dans la revendication 1 ou 2.

4. Utilisation d'une molécule d'acide nucléique codant pour l'inhibiteur selon l'une quelconque des revendications 1 à 3 pour la préparation d'une composition pharmaceutique destinée au traitement de la maladie du greffon contre l'hôte, des maladies auto-immunes, des maladies allergiques, des maladies infectieuses, de la sepsie, au traitement des tumeurs, à l'amélioration de la cicatrisation ou à l'induction ou au maintien d'une insensibilité immunitaire chez un sujet.

5. Utilisation selon la revendication 4, dans laquelle ladite molécule d'acide nucléique est comprise dans un vecteur.

6. Procédé d'identification et d'obtention d'un agent susceptible d'interférer avec l'interaction entre la TIRC7 et son ligand comprenant les étapes de:
(a) test d'une collection de (poly)peptides ou de substances pour leur capacité à inhiber l'interaction entre la TIRC7 et des (poly)peptides de chaîne du HLA de classe II ou un ou des fragments de ceux-ci en utilisant un système de lecture approprié ; et
(b) identification de (poly)peptides ou de substances qui sont testés positifs pour l'inhibition de l'interaction de la TIRC7 avec son ligand à l'étape (a).

7. Procédé selon la revendication 6, dans lequel lesdits (poly)peptides de chaîne du HLA de classe II ou un ou des fragments de ceux-ci comprennent une séquence d'acides aminés dérivée de SEQ ID N° : 2 ou codée par SEQ ID N° : 1.

8. Procédé selon la revendication 6 ou 7, comprenant en outre l'étape de:
(c) répétition des étapes (a) et (b) avec les (poly)peptides ou les substances identifiés une ou plusieurs fois, dans laquelle le (poly)peptide ou la substance nouvellement identifié remplace les (poly)peptides de chaîne du HLA de classe II en tant qu'appât pour l'identification d'un (poly)peptide ou d'une substance interagissant supplémentaire et est remplacé dans les cycles successifs par le (poly)peptide ou la substance interagissant identifié au cycle précédent.

9. Procédé selon la revendication 8 pour identifier et obtenir un (poly)peptide impliqué dans la régulation de la réponse immunitaire chez un sujet.

10. Procédé d'identification et d'isolement d'un agent destiné au traitement de maladies immunitaires comprenant les étapes de :
(a) criblage d'une cellule hôte comprenant un gène rapporteur dont la transcription est directement ou indirectement activée par des dimères comprenant la TIRC7 et la chaîne du HLA de classe II ou leurs domaines de liaison interagissant correspondants avec un composé à cribler ; et
(b) sélection d'un composé qui réprime l'activation du gène rapporteur.

11. Procédé selon la revendication 10, dans lequel ladite cellule hôte comprend au moins un vecteur d'expression contenant une molécule d'ADN codant pour au moins la chaîne du HLA de classe II ou un fragment de celle-ci fonctionnellement liée à un domaine de transactivation ou de liaison à l'ADN.

12. Procédé selon l'une quelconque des revendications 6 à 11 comprenant en outre:
(a) la modélisation dudit agent ou (poly)peptide par des peptidomimétiques ; et
(b) la synthèse chimique du composé modélisé.

13. Utilisation de la chaîne du HLA de classe II ou d'un fragment ou dérivé de celle-ci dans un procédé selon l'une quelconque des revendications 6 à 12.

14. Procédé de diagnostic in vitro d'un état pathologique ou d'une prédisposition à un état pathologique chez un sujet associé à un trouble qui est médié par ou sensible à l'activité de la TIRC7, comprenant :
(a) la détermination de la présence ou de l'absence d'une mutation dans un polynucléotide codant pour la chaîne du HLA de classe II ; et
(b) le diagnostic d'un état pathologique ou d'une prédisposition à un état pathologique basé sur la présence ou l'absence de ladite mutation,
dans lequel ledit trouble est choisi dans le groupe constitué par la maladie du greffon contre l'hôte, les maladies auto-immunes, les maladies allergiques, les maladies infectieuses, la sepsie, les tumeurs, le défaut de cicatrisation et l'incapacité d'un sujet à provoquer des réponses immunitaires appropriées.

15. Procédé de diagnostic in vitro d'un état pathologique ou d'une prédisposition à un état pathologique chez un sujet associé à un trouble qui est médié par ou sensible à l'activité de la TIRC7, comprenant :
(a) la détermination de la présence ou de la quantité d'expression d'un polypeptide de chaîne du HLA de classe II dans un échantillon biologique ; et
(b) le diagnostic d'un état pathologique ou d'une prédisposition à un état pathologique basé sur la présence ou la quantité d'expression du polypeptide,
dans lequel ledit trouble est choisi dans le groupe constitué par la maladie du greffon contre l'hôte, les maladies auto-immunes, les maladies allergiques, les maladies infectieuses, la sepsie, les tumeurs, le défaut de cicatrisation et l'incapacité d'un sujet à provoquer des réponses immunitaires appropriées.

16. Utilisation d'une trousse dans un procédé selon l'une quelconque des revendications 6 à 15, ladite trousse comprenant un polypeptide de chaîne du HLA de classe II ou un fragment biologiquement actif de celui-ci, une molécule d'acide nucléique codant pour la chaîne du HLA de classe II ou une molécule d'acide nucléique d'une longueur d'au moins 15 nucléotides hybridant avec un gène de chaîne du HLA de classe II, ou un anticorps anti-chaîne du HLA de classe II, et éventuellement des moyens de détection appropriés.
